# EUROPEAN PATENT APPLICATION

(11) **EP 1 857 556 A1**
(43) Date of publication of application: **21.11.2007**
(21) Application number: 06290811.6
(22) Date of filing: 18.05.2006
(51) Int. Cl.: C12N 15/82, C12N 9/24, C12P 19/18

(54) **Genetically engineered Pseudomonas fluorescens strains as biosensor for trehalose detection and quantification in a sample**

(71) Applicant: INSTITUT NATIONAL DE LA RECHERCHE AGRONOMIQUE (INRA), 75007 Paris (FR)
(72) Inventor: Sarniguet, Alain, 35590 Le Rheu (FR); Frey-Klett, Pascale, 54110 Remereville (FR)
(74) Representative: Colombet, Alain André

(57) **Abstract**

The invention relates to methods that detects qualitatively and quantitatively trehalose with engineered *Pseudomonas fluorescens* strains. The strains are *Pseudomonas fluorescens* strains which comprise a reporter gene inserted in *treA* gene of operon trehalose, and in which *treA* expression is specifically inducible by trehalose

SEQUENCE LISTING

<110>  INRA

<120>  Genetically engineered Pseudomonas fluorescens strains as
       biosensor for trehalose detection and quantification in a sample

<130>  BET 05P0983

<160>  2

<170>  PatentIn version 3.3

<210>  1
<211>  4487
<212>  DNA
<213>  Pseudomonas fluorescens

<220>
<221>  misc_feature
<222>  (1)..(4487)
<223>  operon trehalose of strain ATCC 17400

<220>
<221>  misc_feature
<222>  (34)..(738)
<223>  tre R CDS

<220>
<221>  misc_feature
<222>  (1055)..(2497)
<223>  tre P CDS

<220>
<221>  misc_feature
<222>  (2616)..(4262)
<223>  tre A CDS

<400>  1
gccggggttg ttgtgggggg caggggtaga tagttagcgc cgcgcaatat ccgaaaagta      60

aaacttatcc aatgtatgcc gcgactcggt gtactcgaac tgccgcccat cctgcaaaaa     120

cgtctggtta ctcaccacaa tcacatggct ctggccgtcg aggtccaagt gcgcctggtc     180

gtccttgctg cggggcaggg cttcgatggt gcgctgggcg tagctgatct gcagttgcag     240

cgtctgctcg ataaacgcgt agatcgactg ttcggcaatc gtctggtcca ggcccgggat     300

caggtcggcg acgaagcggt tgatgtcgag gatcacccgt ttgccgccga tgcgccgcac     360

tcgcttgatg cgcgtgatca gggtgccggg ctcggcctcg atatgctgct gcagggagcc     420

ttccaggggc agttgagtga attcgaccac ttcggtacgt acgtcatcgc ccaggtcggc     480

gtgggtttcg tggaagctga caatgccccc cagttggaac tcaatcgggt tgggggacag     540

cacgaacgtg cccttgccat ggatcttttg cgcgaaccca cgctcttgca attgctcgat     600

ggcccgacgc acggtgccac ggctggcctg gtaggcgtcc atcaattcgg tttcagaggg     660

aaggcgcgtg ccgcgttgca ggcgttcggt ggtgatgctg gcaagcagat ccgtatagat 720

ctggttgtat ttgctcatgg ggatggctct gtgcccgact tgatcattca aggcaggaac 780

cttagtggca caggtggagt ttgtccattg gaccctcgga tatcgccaat ccctgtagga 840

gcgagcttgc tcgcgaaaaa cgtcaacgat aacgcgaaaa accagacacc ccgcgctgcc 900

ctcaggttct tcgcgagcaa gctcgctcct acaaaagagg tcatcggata tttttgtaac 960

tcgtacagac gagttgttgc tttaactcgt ctgtacgagt acttttgccc tcggcgtgct 1020

gccaataaca aaaatctaaa gtggaagaac aagcatgagc cacgactatt ccaacattgc 1080

ccgcgagatc ctcgaacatc tcgggggcag cgacaacctt gagcaagcgg cccactgcgt 1140

gacgcgtctg cgcctggcgc tcaaggaccc aagcctggtc aacgccagtg cattgaacca 1200

ggtcgatctg gtcaaaggct cgttcttcac cggcggcctg ttccaggtcg tcatcggccc 1260

tggcgaagta gaaaaggtct acgccgccct gcgcgaacag accggccttg ccgccgccac 1320

catcgccgat gtgaagaaga agggcgccga caagaccaac gccctgcagc gcctggttcg 1380

cgtgttctcc gacgtgttca tgccgatcct gccggcgctg atcattgccg gcctgctgat 1440

gggcatcaac aacctgatgg gcgccaaagg catgttcatc gagggccaga ctttgctgga 1500

ggcctatccg aacctggatg gcctgtggag cctgatcaac ctgatggcca acacctcgtt 1560

cgtgttcctg ccggcgctgg tgggctggtc ggcggccaag cgttttggcg gcagtgaaat 1620

cctcggcatc gtactcggcc tgatgctggt gcacccggac ctgctcaacg cctggaacta 1680

cggcaaggcg gtggccggcc tcgacggcca gagcctgccg tacttcgata tttttggctg 1740

gttccagatc gagaaagtgg ggtatcaggg ccagatcctg ccgatcctga tggccgccta 1800

tgtcatgagc gtgattgaga agtggctgcg ggcgcgcgtg cccaatgcca tccaattgct 1860

cgtggtgccg atcaccacca tcgtcgtcac cggcgtgctg gccctggcga ttatcggccc 1920

ggtcacccgt cacctcggca tcctgatcac cgaaggcgtc gtcacgttat tcgacctggc 1980

gccgatggtc ggcggggcga ttttcggcct gctgtatgcg ccgctggtga tcaccggcat 2040

gcaccacatg ttcctcgccg tggacctgca actgatctcg acccagggcg gcacatttat 2100

ctggccgatg atcgtcatgt ccaacctggc ccagggcagt gccgcgctcg gagtgttcta 2160

catgagccgc aatgcacggg acaaaagcat ggcctcgacc tcggcgattt ctgcctactt 2220

cggcatcact gaaccggcga tgtttggggt gaacctgcgc ttcaagttcc cgttctatgc 2280

cgcgttgctc ggctcggcgc tgggcagcat tttcctctca ctgaacaagg tccaggcctc 2340

ggccatcggc gtcggtggtt tgcctgggtt tatctcgatc atcccgcagt acatcccaag 2400

cttcgtcatc ggcatggtta tcgcgattgt ggtgccgttt gttttgacct gtgggttgag 2460

catgaagatc gtgcggccgg gttatcgcgt cgcctgatcg atcgctttcg caggcaagcc 2520

agctcccaca gggggatgca tttcaaatgt gggagcgggc ttgcccgcga agaggcccgc 2580
aaggccagca cataactcta ctgaaggaac ccaccatgca agactggcaa cactcggtga 2640
tctaccagat ctacccaaaa agcttccaca gccatgcggg taacgccacc ggtgacctgc 2700
tgggcatcgt ggacaaactc gattacctgc aatggctggg tgtcgattgc ctgtggatca 2760
ccccgttcct gcgctcgccc cagcgcgaca acggctacga catcagcgac tactacgcca 2820
tcgatcccag ctacgggacc atggccgact gcgacctgct gatcggcgaa gccgccaagc 2880
gcgggatcaa gctgatgctc gacatcgtgg tcaaccacac ctccatcgag cacgaatggt 2940
tccagcaggc gcgcagcagc ctcgacaacc cgtaccgcga cttctacatc tggcgcgatc 3000
agccgaacaa ctgggagtca aagttcggcg gctcggcctg ggaatacgag gcgcagaccg 3060
gccagtactt cctgcacctg ttcgaccaca cccaggccga cctcaattgg gacaacccca 3120
aggtgcgcgc cgaagtctac aaactcatgt gcttctggcg tgacaaaggc gtgggcggtt 3180
tccgcctgga cgtgatcaac ctgatttcca agccggcgga ctttcccgaa gacaacagcg 3240
atggccggcg cttctacacc gatggtccgc acgtgcacga gtacttgcag gaaatgcacc 3300
gcgaagtgtt cgaaggccac gacctgatca acgttggcga gatgtcatcc accagcctcg 3360
agcactgcat tcgctactcg cgcccggagt cgaaagaact gtcgatgacc ttcaactttc 3420
atcatctgaa agtggattac cccaacctgc aaaaatgggt gcgcgccgac ttcgacttcc 3480
tgcaactcaa gcagattctc tctgactggc aactgggcat gcaggccggt ggcgggtgga 3540
atgcgctgtt ctggtgtaac catgaccagc cgcgggtggt ctcgcgattt ggtgatgacg 3600
gcgagtaccg cgtggtctcg gccaagatgc tcgccaccgc cttgcacttt ctgcagggca 3660
cgccgtttgt gtaccagggc gaagagctgg gcatgaccaa tccggggttc gaccagatcg 3720
ggcagtaccg cgatgtcgag accctgaaca tctaccgcct caagcgcgat gcgggcgagt 3780
cgcaagcgtc aagcatggcc gccatcatgc aaaagtcccg ggataacggg cgcacgccga 3840
tgcaatggaa cggccaggca aatgccggct tcagcagcgg tgaaccgtgg attggcatcc 3900
cggccaatgc ggcgcagatc aatgtgcaga gccagttgga tgacccggat tcggtgctgc 3960
atcactaccg ggcgctgatc gccttgcgtc gccttgagcc gctgatccag cagggcgtgt 4020
accgccaact gttgtgcgat cacgccaaag tctgggcgta tctgcgtgaa ggccatggcg 4080
agcgcctgct ggtgctgaac aacttctacg gccagccctg cgagatccaa ctgcctggcc 4140
aagtgatcag cgcggcgagc gcgcaacggc tgctgatcag caactacccc gactgcccgg 4200
tgcgcaccga cagcatcacc ttgcgcccat acgagtcttt tgtgctgcac ctgaccgact 4260
gacattcccc tttttgtagg cgcagcttgc tcgcgaaaaa cttcaaagcg ccgcgtgcat 4320

ccgcaatgcc cacgtcaccg ttgacgattt tcgcgagcaa gctcagactc ctacagaatc 4380

gcgttacaca caataagaaa aaaggagcgc ttcatgaaaa caacaataaa gctgggcctc 4440

gctgcatcct gtctgacgtt gcccctggcg gcccacgccc tggaatt 4487

<210> 2
<211> 2103
<212> DNA
<213> Pseudomonas fluorescens

<220>
<221> misc_feature
<222> (1)..(2103)
<223> TreA gene of strain BBc8R8

<220>
<221> misc_feature
<222> (1362)..(1363)
<223> SphI restriction site

<400> 2
tgatcgtcat gtccaacctg gcccagggta gcgcggcgct cggcgtgttc tacatgagcc 60

gcaatgcgcg ggacaagagc atggcgtcca cctcggccga tttccgccta cttcggcatc 120

accgagccgg cgatgttcgg ggtcaacctg cgcttcaagt ttccgttcta cgcggcgctg 180

accggctcgg cgctgggcag tattttcctc tcgctgaaca aggtgcaggc ctcggcgatt 240

ggcgtgggtg gcttgcccgg gttcatctcg atcatcccgc agtacatccc aagttttgtc 300

atcggcatgg tcgtcgcgat tgtcgtgccg tttgttttga cctgtgtgtt gagcatgaaa 360

attgtccggc ctggctaccg ggtcgcctga tctaccgcta tcgcaggcaa gccagctccc 420

acagttgatc gcatttccag attgaaggaa cccgtcatgc aaaactggca acattcggtg 480

atctaccaga tctaccccaa aagcttccac agccacgcgg gtaacgccac cggtgacctg 540

ctgggcatcg tggacaagct cgattacctg aagtggctgg gcgtcgactg cctgtggatc 600

accccgttcc tgcgttcgcc ccagcgcgac aacggctacg acatcagtga ctactacgcc 660

atcgacccga gctacgggac catggccgat tgcgacctgc tgatcagcga agccgccaag 720

cgcggcatca cgctgatgct cgacatcgtg gtcaaccaca cctccatcga acacgagtgg 780

ttccagcagg cccgcagcag cctcgacaac ccgtaccgcg acttctacat ctggcgcgac 840

cagccgaaca actgggaatc caagttcggc ggctcggcct gggaatacga ggcgcagacc 900

ggccagtact tcctgcacct gttcgaccac acccaggccg acctgaactg ggacaacccc 960

aaggtgcgcg ccgaagtgtt caagctgatg cgcttctggc gcgacaaggg cgtgggcggt 1020

ttccgcctgg acgtgatcaa cctgatctcc aagccggcgg attttccgga agacaacagc 1080

gacggtcggc gcttctacac cgacggcccg aacgtgcatg aatacctgaa ggaaatgcac 1140

cgcgaagtgt tcgaggggca tgacctgatc aacgtcggcg agatgtcgtc caccagcctt 1200

gagcactgca ttcgttattc gaatccacag tcgaaagagc tgtcgatgac cttcaacttt 1260

caccacctga aagtcgatta tccgaacctg cagaagtggg tcgcggccga cttcgacttc 1320

ctgcaactca agcagattct gtccgactgg caactcggca tgcaggccgg tggcggctgg 1380

aatgcgctgt tctggtgtaa ccacgaccag ccgcgggtgg tctcgcgttt tggtgacgac 1440

ggcgagcacc gcgtggtctc ggcgaagatg ctcgccactg cgctgcactt cctccagggc 1500

acgcctttcg tgtaccaggg cgaagagttg ggcatgacca atccggcttt cgacaccatc 1560

gaccaatacc gggatgtcga gacgctgaac atctatcggc tcaagcgtga ggccggtgag 1620

tccgaggcat cgagcatggc ggcgatcatg cagaagtccc gcgataacag ccgcacaccg 1680

atgcaatgga atgccgagca aaacgccggc ttcagccgcg gcgaaccgtg gatcggcatc 1740

ccggccaacg ccgcgcagat caacgtgcaa agcgagctgg atgacccgca atcggtactg 1800

catcactacc gtgcgctgat caagctgcgt cggcatgagc cgctgattca ggaaggcgtt 1860

tatcgccaac tgctgccaca gcacacccag gtctgggcgt acctgcgcga aggccacggc 1920

gaacgcctgc tggtgctgaa caacttctac ggcacgccat gccagatcga actgcccgat 1980

ggcctgctga cggcggcgag tgagcaacgc ctgctgatca gcaactacga cgactgccca 2040

cagcgctcga gccaggtctc gctgcgcccg tatgaatcgt ttgtgttgca cctgaccgac 2100

tga 2103

## Description

The invention relates to methods that detect qualitatively and quantitatively trehalose with engineered *Pseudomonas fluorescens* strains. The strains are *Pseudomonas fluorescens* strains that comprise a reporter gene inserted in *treA* gene of operon trehalose, and in which *tre*A expression is specifically inducible by trehalose.

Trehalose (CAS 6138-23-4 (dehydrate)) is a naturally occurring disaccharide containing two glucose molecules bound in an α,α-1,1 linkage. Trehalose is encountered in numerous organisms like fungi, bacteria, nematodes, insects and mammals (Elbein A. D., 2003). Because trehalose is sometimes accumulated in high concentrations in cells or in specific organs, it serves as a carbohydrate reserve that can be quickly metabolised. Many physical properties are conferred to this nonreducing sugar: a high hydrophilicity, a chemical stability, nonhygroscopic glass formation and the absence of internal hydrogen bond formation (Argüelles, 2000). That is why trehalose is considered as a stress metabolite (Attfield, 1987 ; Fillinger S. 2001), which most often acts as an osmoprotectant (Boos et al., 1990) and as a cold protectant (Krandror et al., 2002). As trehalose increase protein stability (Colaco *et al*., 1992; Kaushik and Bhat, 2003), it is now used for DNA engineering (Carninci et al., 1998), cryoprotection (Eroglu *et al.,* 2002), and food conservation (Paiva and Panek, 1996).

Despite numerous studies about biochemical and biological roles of trehalose, its involvement in physiology and also in organism interactions continues to be explored.

In plants, only few species, mainly desiccation-tolerant ones, were considered to accumulate trehalose until recently (Wingler, 2002). However, the discovery of trehalose-6-phosphate synthase and trehalase-6-phosphate phosphatase genes in *Arabidopsis* genome (Blasquez *et al.,* 1998 ; Vogel et al., 1998) suggests that the ability to synthesize trehalose may be widely distributed in the plant kingdom (Müller et al., 2001), although most plant species do not appear to accumulate easily detectable amounts of trehalose. It nevertheless accumulates in specific plant organs like senescent nodules from leguminous plants-*Rhizobium* symbiosis (Streeter, 1985 ; Mellor 1988 ; Muller *et al.,* 2001), and in clubroots from *Arabidopsis-Plasmodiophora brassicae* pathogenesis (Keen and Williams, 1969 ; Broadmann *et al*., 2002).

The possible involvement of trehalose in plant-microorganism interactions has recently given rise to the investigation of new biological roles for this sugar. If trehalose seems to be exchanged between organisms in eukaryote-eukaryote and eukaryote-prokaryote interactions, it has not yet been proved how much trehalose is really accumulated especially in fungi other than yeast and mycorrhizal fungi. The main methods which were developed to quantify trehalose rely on the assessment of natural or artificial ¹³C abundance by NMR analysis (Martin et al., 1984 ; Dijkema C et al., 1985 ; Bellinger and Larher, 1988 ; Meikle et al., 1991), on high-performance chromatography separation and detection of carbohydrates (Cataldi et al., 1999 ; Shi et al., 2002), or on indirect assessment by measuring *in vitro* trehalase activity (Araujo et al., 1989 ; Kienle et al., 1993). Enzymatic assays have also been developed, for instance based on trehalose hydrolysis to D-glucose by trehalase, then conversion of D-glucose into glucose-6-phosphate (G-6-P) by hexokinase and subsequent oxidation of G-6-P with simultaneous formation of NADPH, NADPH being measured at last (Trehalose Assay Kit, Megazyme, Ireland). However, such assays do not make it possible to measure trehalose directly while avoiding glucose interferences. But glucose is almost always present in biological and food samples. So, most of these methods are cumbersome, expensive, not sensitive enough or difficult to adapt to the analysis of small and numerous samples.

Beside these classical methods, new tools have emerged with the use of reporters genes (Leveau and Lindow, 2002). Indeed, some biosensor bacterial strains have been constructed that indirectly assess the transcription level of microbial genes involved in sugar transport and catabolism (Miller et al., 2001). They can be used to monitor carbon availability in various environments such as the plant rhizosphere (Jaeger et al., 1999 ; Standing et al., 2003). However only very few biosensors were constructed which are specific for carbohydrates (Hansen and Sorensen, 2001 ; Miller et al., 2001).

The trehalose synthesis and regulatory pathways have been described in several bacteria like *Escherichia coli* (Boos et al., 1990), *Bacillus* (Helfert et al., 1995), *Sinohizobium meliloti* (Jensen et al., 2002), and *Pseudomonas* (Matthijs et al., 2000).

The inventors have developed a biological method to quantify trehalose concentrations. It is based on the specific induction of the phosphotrehalase gene *treA* of *P*. *fluorescens.* Thanks to its simplicity and sensitivity, the method allows the analysis of small and numerous samples.

Many applications can be developed with this method of quantification. For instance, species, in particular phytopathogenic fungal species, may be compared according to their ability to accumulate trehalose. Furthermore, the method may be used to identify the optimal environmental conditions that are required for the trehalose accumulation in cells, and especially the improvement of the quality of microbial inoculums.

### Definitions

*"Pseudomonas fluorescens"* encompasses numerous root and mycorrhiza-associated microorganisms. Many *P*. *fluorescens* strains are publicly available. Non limitative examples of *P*. *fluorescens* strains include the strain deposited at the American Type Culture Collection (ATCC) under number 17400, as well as *P*. *fluorescens* Pyt 6, Pf29A and BBc6 strains which were isolated respectively from wheat roots infected with the *Gaeumannomyces* graminis fungus (Chapon et *al.* 2002) and ectomycorrhizae of Douglas fir*-Laccaria bicolor* (Garbaye and Duponnois, 1992). The *Pseudomonas fluorescens* Pyt 6 strain has been deposited before the deposit authority CNCM (Collection Nationale de Cultures de Microorganismes, Institut Pasteur) under number CNCM I-3571 on 10 February, 2006.

The trehalose operon of *Pseudomonas fluorescens* has been characterized (Gaballa et al., 1998; Matthijs et al., 2000). In P. *fluorescens* strain ATCC 17400, the trehalose operon contains at least three genes *treP, treA* and *treR, treR* encoding a putative repressor protein for *treP* and *treA* transcription. Extracellular trehalose inhibits *treR,* and allows *treA* and *treP* transcription (Matthijs et al., 2000). The gene *treP* codes for a putative enzyme II subunit of the phosphotransferase system that catalyzes the phosphorylation of trehalose together with its translocation across the cell membrane and *treA* encodes a putative phosphotrehalase, which hydrolyzes the incoming trehalose-6-phosphate into glucose and glucose-6-phosphate. Both genes are negatively regulated by TreR, a repressor of the FadR-GntR family of transcription regulators. The sequence of trehalose operon of strain ATCC17400 has been submitted to Genbank under accession number AF229829 (SEQ ID N0:1).

A "reporter gene" denotes a gene which upon expression produces a gene product (i.e. protein) which is detectable with appropriate means. Reporter genes can be attached to other sequences so that only the reporter protein is made or so that the reporter protein is fused to another protein (fusion protein). Commonly used reporter genes include for instance *lac Z* which encodes β-galactosidase, *lux* which encodes luciferase, and *gfp* which encodes green fluorescent protein (GFP).

As used herein, the term "specifically inducible by trehalose", when referring to *treA* gene expression, means that *treA* expression is triggered by trehalose but not by another sugar, in particular either of, or preferably each of glucose, maltose, fructose, saccharose, arabinose and galactose, up to 0,2% (w/v) concentration in the culture media.

### Genetically engineered Pseudomonas fluorescens strains with trehalose inducible TreA -reporter gene constructs

It is herein described genetically engineered *Pseudomonas fluorescens* strains which comprise a reporter gene inserted in *treA* gene of operon trehalose, and wherein *treA* expression is specifically inducible by trehalose.

Gaballa et al. (1997) have described introduction by transposon mutagenesis of a "lac Z2-Kanamycine" cassette in trehalase gene (*treA*) of *P*. *fluorescens* strain ATCC 17400. A transcriptional fusion was generated between *treA* gene and *lacZ2* gene. In the mutated strains, β-galactosidase is expressed upon the induction of *treA* transcription. One of the 2400 clones thus obtained was further characterized (the so-called strain "Pyt 6"). β-galactosidase ("β-gal" as used hereafter) expression in Pyt 6 was described as inducible by trehalose but also by high osmolarity. Contradictory results were published later by Matthijs et al. (2000) who concluded that, in Pyt 6, *treA* gene was induced by trehalose but not by high osmolarity.

The inventors hypothesised that a strain with a reporter construct inducible by trehalose could comprise of a biosensor useful for assaying trehalose, provided induction is sufficiently specific for trehalose, in particular to avoid interference from sugars such as glucose.

The inventors were kindly provided with three of the *P*. *fluorescens* clones (Pyt 5, Pyt 6 and Pyt 61) developed by the team whose work was published in Gaballa et al. (1997) and Matthijs et al. (2000).

The inventors assayed these strains for trehalose specific *treA* induction and observed that Pyt 6 did not display trehalose specific *treA* induction since *treA* expression was also triggered by glucose and maltose. Furthermore Pyt 61 strain did not show any β-gal induction in all tested conditions. On the contrary, the inventors showed that *treA* expression was specifically induced by trehalose in Pyt 5 strain. Therefore these results were conflicting with those published by Matthijs et al. (2000).

In spite of these repetitive discrepancies, the inventors decided to disregard the Pyt 6 and Pyt 61 strains, and selected the Pyt 5 strain for further characterization. They lately elucidated that an inversion had been made in storage tubes of the strains they were sent.

In conclusion, the inventors have shown that not all strains provided by the Flanders Interuniversity for Biotechnology VIB with a Tn*5*-lacZ2-Km cassette inserted in the *treP-treA* operon can provide a basis for trehalose quantification. They demonstrated that the *Pseudomonas fluorescens* Pyt 6 strain referred as Pyt 6 in Gaballa et al. (1997) and Matthijs et al. (2000) and deposited before CNCM (Collection Nationale de Cultures de Microorganismes, Institut Pasteur) under number CNCM I-3571 can be used for specific induction by trehalose and quantitative assessment of trehalose. They have further established that the location of the insertion of the cassette in the *treA* gene is a key factor to get a strain in which reporter gene expression is specifically induced by trehalose. For this, the insertion site of the Tn*5*-lacZ2-Km cassette inserted in Pyt 6 was exactly characterized. The insertion site is defined by nucleotides 933 to 940 of *treA* coding sequence, i.e. by nucleotides 3548 to 3555 of the sequence shown as SEQ ID NO:1 (AF229829 sequence : 4487 bp linear DNA including the trehalose operon of the wild ATCC17400 strain). Accordingly, a target insertion region of about 50 base pairs upstream and downstream this insertion site may be defined, *e.g.* a target region defined by nucleotides 3498 to 3605 of SEQ ID NO:1, preferably defined by nucleotides 3518 to 3585 of SEQ ID NO:1.

It is thought that a gene reporter insertion within this target region of *treA* generally leads to a specific induction of *treA* expression by trehalose. A corresponding region in *P*. *fluorescens* strains which are different from ATCC 17400 may be readily identified by the one skilled in the art, for instance by performing alignment between *treA* sequence of ATCC 17400 and *treA* sequence of a variant strain. In said variant strain, a region "corresponding to" the above target region (or at close vicinity) would be identified as being substantially homologous to nucleotides 933 to 940 +/- 50 nucleotides of the *treA* coding sequence, i.e. by nucleotides 3598 to 3555 +/- 50 nucleotides of the sequence shown as SEQ ID NO:1.

Nucleotide sequences (DNA or RNA) are "substantially homologous" or "substantially similar" when at least 50%, preferably at least 70%, still preferably 80 %, and most preferably at least 90 or 95 % of the nucleotides match (i.e. are identical) over the defined length of the sequences, as determined by sequence comparison algorithms, such as BLAST, FASTA, DNA Strider, etc.

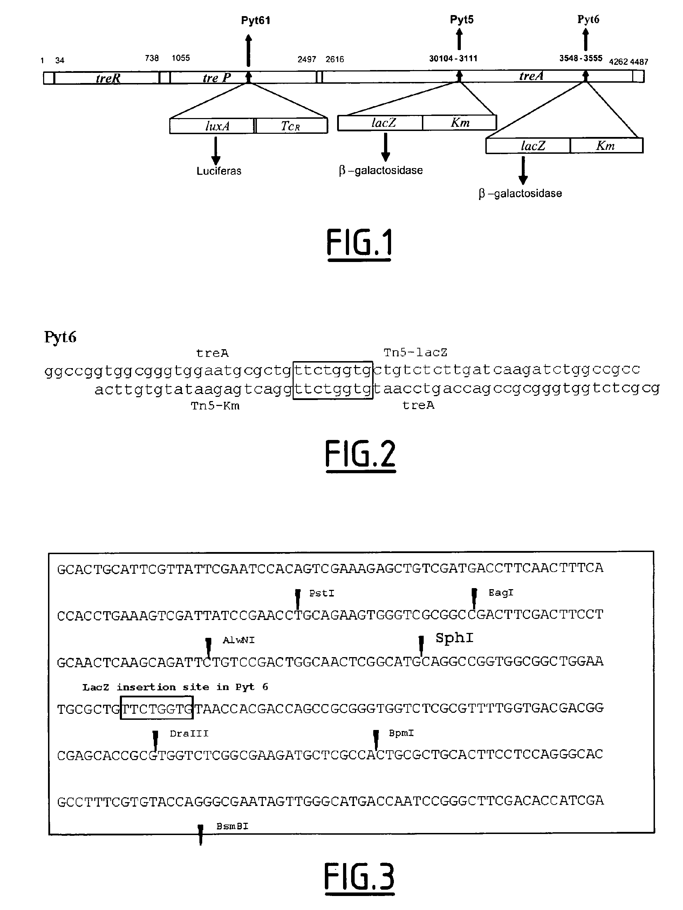

An example of a corresponding target region in strain BBc6R8 may be an insertion site consisting of the Sphl restriction site as shown on Figure 3.

A strategy of saturation of insertion in the *treA* gene (bank of mutants containing a single cassette insertion in multiple positions) could easily provide other mutants suitable for specific and linear induction by trehalose and so specify another useful location for a gene reporter insertion.

Therefore, the invention relates to a genetically engineered *Pseudomonas fluorescens* strain comprising a reporter gene inserted in *treA* gene of operon trehalose, as defined above, and wherein said reporter gene is inserted in a region of *treA* gene homologous to nucleotides 3498 to 3605 of SEQ ID NO:1, preferably nucleotides 3518 to 3585 and still preferably nucleotides 3548 to 3555 of SEQ ID NO:1, with the provisio that said *P*. *fluorescens* strain is not Pyt 6 described by Gaballa et al. (1997) and Matthijs et al. (2000) and deposited before the CNCM under number I-3571.

Preparation of such genetically engineered strains is within the ordinary skills of the one skilled in the art. The *P*. *fluorescens* strains according to the invention may be obtained for instance by site directed mutagenesis or gene exchange.

Non limitative examples of *P*. *fluorescens* strains which may be genetically engineered include ATCC 17400, BBc6R8 (Frey-Klett et al, 1997), Pf29A, and any strain of *P*. *fluorescens* having a trehalase activity (biovar I) according to Fahy and Lloyd (1983).

According to an embodiment said genetically engineered *Pseudomonas fluorescens* strain which comprises a reporter gene inserted in *treA* gene of operon trehalose is a BBc6R8 strain which comprises a reporter gene (e.g. *gfp*) inserted at the Sphl restriction site, i.e. between nucleotides 1362 and 1363 of *treA* sequence, as shown in SEQ ID NO:2.

### Use of genetically engineered Pseudomonas fluorescens strains

Genetically engineered strains, including strain Pyt 6 as described by Gaballa et al. (1998) and Matthijs et al. (2000), were found to be useful for rapid and sensitive detection of trehalose in a sample.

Therefore, the invention further relates to the use of a genetically engineered *Pseudomonas fluorescens* strain which comprises a reporter gene inserted in *treA* gene of operon trehalose, wherein *treA* expression is specifically inducible by trehalose, for assaying trehalose in a sample.

Preferably in the strains which are used, *treA* expression is not inducible by glucose and/or maltose. Also preferably *treA* expression is neither inducible by arabinose and/or fructose and/or galactose and/or saccharose.

While the invention is not limited to this embodiment, it may be preferred that the genetically engineered *P*. *fluorescens* strain comprises a reporter gene inserted in a region of *treA* gene homologous to nucleotides 3498 to 3605, preferably 3518 to 3585, and still preferably 3548 to 3555 of the sequence shown as SEQ ID NO:1.

Said *P*. *fluorescens* strain may be selected from the group consisting of ATCC 17400, BBc6R8, Pf29A strains and other *Pseudomonas* biovar I strains having an endogenous inducible trehalase activity.

For instance, a genetically engineered *P*. *fluorescens* strain "Pyt 6" as described by Gaballa et al. (1998) and Matthijs et al. (2000) or a genetically engineered BBc6R8 strain in which a reporter gene (e.g. *gfp*) sequence inserted between nucleotides 1362 and 1363 of SEQ ID NO:2 may be used.

The sample assayed may be in liquid, semi-solid or solid state.

The samples to be measured may be foods, beverages and other materials, such as microorganisms, eukaryotic cells and tissues, or solutions for preservation of living tissues or cells and any ingredient containing trehalose (*e.g*. cosmetic products, paintings... ).

### Applications of genetically engineered Pseudomonas fluorescens strains

Trehalose has been determined to be Generally Recognized As Safe (GRAS) as a multipurpose ingredient for all uses in food, including as a sweetener, stabiliser, thickener and flavour enhancer. Trehalose is used as a sweetener in food industry, with a relative sweetness as compared with sucrose of 45 %. It is one of the most stable saccharides, trehalose showing high thermostability and a wide pH-stability range. Furthermore trehalose is a non reducing sugar, hence does not show Maillard reaction with amino compounds such as amino acids or proteins. The physical features of trehalose make it an extremely attractive substance for food industry applications.

In another field, trehalose has been considered for effective preservation of organs (Chen et al., 2004) and has been found to show cryoprotective potential for storage of haematopoietic stem cells (Scheinkonig et al., 2004).

Additionally, trehalose is found in many organisms, including bacteria, fungi, insects, plants and invertebrates. In these organisms, trehalose is a protectant against various stresses such as freezing, dryness and osmotic pressure. The measurement of trehalose in these organisms after culturing/breeding in different environmental conditions could allow quantifying accumulation of trehalose and thus identifying the culturing/breeding conditions optimal for subsequent preservation.

Until recently, conventional method of producing trehalose was extraction from yeast. Due to low yields, and high costs, this method has been replaced with enzymatic conversion of maltooligosaccharide (Igashiyama T., 2002). Thus, the use of a genetically engineered *P*. *fluorescens* strain as described above could help identifying organisms, in particular microorganisms such as fungi, which naturally accumulate trehalose and which could prove useful for industrial production of trehalose. The best conditions for optimal accumulation of trehalose could be determined similarly.

These applications are further described in the following methods according to the invention as well as in the figures and examples.

The inventors have successfully used the genetically engineered *Pseudomonas fluorescens* strains described above in an overlay technique to qualitatively detect trehalose in fungal mycelium. In these experiments, mycelium were cut to release trehalose which induced *treA* expression in the *P*. *fluorescens* strains and thereby reporter gene expression. Presence of trehalose is thus revealed by detecting presence and/or activity of the expression product of the reporter gene. Where the reporter gene is beta-galactosidase, should endogenous beta-galactosidase activity exist in the tested organism, the trehalose accumulation can be readily assessed by detecting a higher expression while using the *P*. *fluorescens* strains compared with a control without the *P*. *fluorescens* strains in the overlay. Thus, the strains according to the invention may be used in a simple method of screening of trehalose producing / accumulating microorganisms.

These experiments validate the use of genetically engineered *P*. *fluorescens* strains as a biosensor for detecting trehalose in a sample.

The invention thus relates to a method of qualitatively detecting trehalose in a sample, said method comprising the steps consisting of:
a) contacting said sample with a genetically engineered *Pseudomonas fluorescens* strain which comprises a reporter gene inserted in *treA* gene of operon trehalose, wherein *treA* expression is specifically inducible by trehalose, as defined above;
b) detecting presence and/or activity of an expression product of the reporter gene; and
wherein presence and/or activity of the expression product of the reporter gene is indicative of the presence of trehalose in the sample.

The sample may be a food or beverage sample, as well as an eukaryotic cell, tissue or microorganism such as a fungus, a yeast, a bacteria and any ingredient containing trehalose (*e.g*. cosmetic products, paintings...).

Contacting of the sample with the *P*. *fluorescens* strain may be performed in liquid or semi-solid phase, as appropriate. Contacting is performed under conditions and for a time appropriate to allow expression of the reporter gene, where the *P*. *fluorescens* strain is contacted with trehalose.

Where the expression product of the reporter gene is likely to be endogenously produced or present in the sample (for instance where the reporter gene is β*-gal* and the sample is a microorganism), a control experiment may be performed by detecting the level and/or activity of the expression production of the reporter gene in a sample which has not been contacted with the genetically engineered *P*. *fluorescens* strain. If an increased level and/or activity of the expression product of the reporter gene is detected in the sample contacted with the *P. fluorescens* strain, as compared with the control sample which was not contacted with said strain, then this increased level and/or activity of the expression product of the reporter gene is indicative of the presence of trehalose in the sample.

In step b), depending on the reporter system used (i.e. reporter gene, and means of detection of reporter gene expression product or activity), a lysis of *P*. *fluorescens* cells may be advantageously performed to release the reporter gene expression product and facilitate detection of its level and/or activity.

According to an embodiment said method of qualitatively detecting trehalose is applied for identifying microorganisms which accumulate trehalose. Said method may comprise the steps consisting of:
a) contacting a candidate microorganism with a genetically engineered *Pseudomonas fluorescens* strain which comprises a reporter gene inserted in *treA* gene of operon trehalose, wherein *treA* expression is specifically inducible by trehalose, as defined above; and
b) detecting the presence and/or activity of the expression production of the reporter gene;
wherein presence and/or activity of the expression production of the reporter gene is indicative of the accumulation of trehalose by the microorganism.

While not compulsory, to facilitate detection of trehalose, the microorganism may be cut, crushed, ground, etc, where appropriate, in order to release trehalose contained therein. The one skilled in the art may refer for instance to the method of fungal extraction described in the following examples.

Contacting of the microorganism with the genetically engineered *Pseudomonas fluorescens* strain may be carried out for instance in semi-solid phase, by culturing the microorganism in agar and pouring above a layer of agar medium containing the P. *fluorescens* strains, as detailed in the overlay method described in example 2 below.

The inventors further developed a method of quantifying trehalose in a sample which comprises the steps consisting of:
a) contacting said sample with a genetically engineered *Pseudomonas fluorescens* strain which comprises a reporter gene inserted in *treA* gene of operon trehalose, wherein *treA* expression is specifically inducible by trehalose;
b) measuring the level and/or activity of an expression product of the reporter gene; and
c) comparing the level and/or activity measured in step b) with the level and/or activity of the expression product of the reporter gene elicited by a range of trehalose standards of known concentrations, to determine trehalose concentration in the sample.

Preferably, the method is carried out in solution. The method can be carried out in small volumes, for instance in 96-wells microtitration plates, and thus can be automated.

The sample may be a food or beverage sample, as well as an eukaryotic cell, tissue or microorganism such as a fungus, a yeast, a bacteria, or an extract of a microorganism or any ingredient containing trehalose (*e.g.* cosmetic products, paintings...). Where the sample is not in liquid state, an extraction, or any other appropriate treatment known by the one skilled in the art, may be performed on the sample to render trehalose accessible.

Contacting of a liquid sample with a genetically engineered *Pseudomonas fluorescens* strain may be carried out by mixing a suspension of *P*. *fluorescens* cells with the sample.

The particular conditions under which contacting of the sample with the genetically engineered *P*. *fluorescens* strain may be performed can be readily identified by the one skilled in the art. The one skilled in the art may refer for instance to the adapted media and method developed by inventors. These conditions may depend on the particular *P*. *fluorescens* strain chosen for carrying out the method of the invention.

Examples below illustrate adjustments that may be made on the sample (e.g. pre-treatment of the sample, concentration) and/or on the *P*. *fluorescens* strain (e.g. cell density, growth phase, culture medium, etc) to optimize sensitivity of the method of quantifying trehalose.

For instance, under certain conditions, it may be preferred that the sample be contacted with cells of a genetically engineered *P*. *fluorescens* strain which are in exponential growth phase.

Also, for example, where the sample is a fungal mycelium extract, and depending on the reporter system selected, it may be preferred that the extract be boiled and that the extract be diluted, for instance to 1/10, before contacting with the *P. fluorescens* strain.

Preferred examples of genetically engineered *P*. *fluorescens* strains to be used in the method of qualitative and quantitative determination of trehalose include "Pyt 6" as described by Gaballa et al. (1997) and Matthijs et al. (2000) (deposited before CNCM under number I-3571) or BBc6R8 strain with a reporter gene (e.g. gfp) inserted at the Sphl restriction site of *treA* gene.

Where the genetically engineered *P*. *fluorescens* strain is Pyt 6, a buffered medium, such as a buffered casamino acid (BCAA) medium, of pH ranging from 6.9 to 7.2 may be used for trehalose quantification. It may be a BCAA (Bacto Casamino Acid (Beckton, Diskinson and Company) 0.5 g, MgSO₄·7H₂O 0.025 g, H₂O 90 ml sterilized by autoclave at 115°C, 20 min +10 ml of Potassium phosphate buffer (pH 7)) solution, or a BCAA 0.5% agar medium (+ Agar 2.0 g), optionally containing 0.1 mM FeSO₄ and 0.2 % glucose.

The inventors further found that a time length ranging from 3 to 4 hours, preferably about 3 hours, at a temperature ranging from 20°C to 27°C, preferably about 27°C, proved to be appropriate for contacting of Pyt 6 cells with the sample, in particular where O.D._{600 nm} of a suspension of Pyt 6 cells is adjusted to about 1.0 and said contacting performed by diluting said suspension to ½ with the sample. However, other contacting conditions may be used and identified by the one skilled in the art.

Thus, according to an embodiment, step a) of the quantitative method is performed by mixing the sample, optionally previously diluted, with a suspension of Pyt 6 cells in a buffered medium with a pH ranging from 6.9 to 7.2 and incubating the mixture at a temperature ranging from 20°C to 27°C, for a time length of 3 to 4 hours.

With Pyt 6 strain, trehalose is quantified by measuring β-galactosidase activity. Since fungi may produce β-galactosidase endogenously, where the sample is a fungal mycelium extract, it is preferred that the extract be boiled and diluted before contacting with the genetically engineered *P*. *fluorescens* strains, in particular Pyt 6 strain or any other strain wherein the reporter gene is i*β-gal.* As described in example 3, a ten-fold dilution, for instance, is an appropriate dilution for improving sensibility of the method of the invention.

The choice of a particular reporter system (i.e. reporter gene and appropriate means of detection of the reporter gene expression product or activity thereof) may be governed by the luminescent/fluorescent properties of the sample to be measured, or by the likelihood of interference from endogenous components of the sample with the signal associated with the reporter gene expression product (for instance endogenous production of β-galactosidase by fungi where the reporter gene is β*-gal*)*.*

Examples of reporter systems which may be used include the reporter gene β-gal, β-galactosidase being quantified by measurement of signal associated with processing of substrates such as the chromogenic X-gal, or preferably the chemiluminescent Galacto-Star substrate from Applied Biosystems. Alternatively, *gfp* may be used as a reporter gene, its expression product (GFP) being quantified by fluorescence measurement.

Hence, according to an embodiment, step b) of the method of quantifying trehalose may be carried out by mixing an aliquot of the mixture of said sample and P *fluorescens* strain of step a) with a chromogenic or chemiluminescent substrate of β-galactosidase and measuring by colorimetry or luminescence, as appropriate, the signal generated by the processed substrate.

According to another embodiment, step b) of the method of quantifying trehalose may be carried out by measuring GFP fluorescence in the mixture of said sample and *P fluorescens* strain of step a).

In step b), depending on the reporter system used, a lysis of *P*. *fluorescens* cells may be advantageously performed to release the reporter gene expression product before measuring of its level and/or activity.

The method of quantifying trehalose according to the invention may also be semi-quantitative, for instance where the level and/or activity of the expression product of the reporter gene is compared with that elicited by a single trehalose standard of known concentration, or where comparison with the level and/or activity of the expression product of the reporter gene elicited by a range of trehalose standards of known concentrations is only performed to determine trehalose concentration in the sample is above a threshold level.

For instance, the Inventors have developed a semi-quantitative method of assaying trehalose in a sample. Said method comprises the steps consisting of:
a) contacting the sample with a suspension of Pyt 6, precultured to exponential growth, at OD 600 nm = about 0.1, with a ratio about 1:20 (preferably 10 µl of the sample, optionally diluted, are contacted with 190 µl of the Pyt 6 suspension atOD600nm=0.1);
b) adding about 2 µl X-Gal (20 mg/ml);
c) incubation the mixture during 16-20 hours, preferably18 hours, at 27°C (preferably with agitation at 150 rpm),
c) observing and/or measuring the blue coloration elicited by X-Gal processing; and
d) comparing the blue coloration observed in step c) with that elicited by a standard of trehalose contacted with a suspension of Pyt 6, precultured to exponential growth, at OD 600 nm = about 0.1, with a ratio 1:10 (preferably 10 µl of the standards are contacted with 190 µl of the Pyt 6 suspension at OD 600 nm = 0.1) and X-Gal (2 µl, 20 mg/ml);
whereby if the blue coloration observed in step c) is above that of observed with the standard, then the concentration of trehalose in the sample is above trehalose concentration in the standard.

With the conditions defined in the above method, the choice of a standard with 0.5 mM trehalose enables for a reliable detection of trehalose in samples. The threshold level is then 0.5 mM trehalose.

A range of trehalose standards may also be used in order to provide for an even more accurate comparison of the blue coloration obtained with the sample and with the trehalose standard which defines the threshold level. For instance, with a threshold level of 0.5 mM, trehalose standards ranging from 0.1 mM to 2 mM can be used.

The method of quantifying trehalose according to the invention may be applied for screening trehalose accumulating microorganisms and in particular identifying those with higher rates of trehalose accumulation. This may be of interest for identifying candidate microorganisms useful for producing trehalose on industrial scale.

Thus, the invention further relates to a method of screening trehalose accumulating microorganisms, which method comprises the steps consisting of:
a) assaying trehalose in a candidate microorganism by a method of trehalose detection and/or quantitation as described above; and
b) identifying as a microorganism which accumulates trehalose, the microorganism in which trehalose is detected.

Preferred these candidate microorganisms include those in which trehalose production has been reported, e.g. yeasts, fungi, or bacteria.

Said method of screening may be implemented for identifying microorganisms useful for producing trehalose on industrial scale.

Thus, the invention also relates to a method of screening trehalose accumulating microorganisms useful for producing trehalose on industrial scale, which method comprises the steps consisting of:
a) quantifying trehalose in candidate microorganisms by a method of trehalose quantification as described above; and
b) identifying as useful for producing trehalose on industrial scale that(those) microorganism(s) in which the highest quantity(ies) of trehalose is(are) detected.

All candidate microorganisms in which trehalose is to be detected may have been cultured under identical environmental conditions, or each microorganism may have been cultured under environmental conditions identified as optimal for trehalose accumulation in that particular microorganism.

The microorganisms in which the highest quantity(ies) of trehalose is(are) detected may be identified by ranking the tested microorganisms according to decreasing quantities of trehalose measured, and selecting the first 20 %, or preferably first 10 %, of strains with the highest trehalose quantities.

The invention further provides a method of producing trehalose which comprises : culturing a microorganism, preferably under conditions optimal for trehalose accumulation, and recovering the trehalose produced by the microorganism. Preferably, said microorganism has been identified as useful for producing trehalose on industrial scale.

Additionally, the method of quantifying trehalose according to the invention may be advantageously used to quantify trehalose accumulation in a microorganism exposed to different culture conditions, and thereby help identifying culture conditions allowing optimal trehalose accumulation. This may be of interest for improving trehalose yields obtained by culturing microorganisms in view of industrial production of trehalose. Additionally, since trehalose is regarded as preserving cells from freezing and osmotic stresses, storage of cells or microorganisms could be improved by culturing the cells or microorganisms to be cryopreserved or lyophilised (for instance) under conditions identified as optimal for trehalose accumulation.

Thus, the invention also relates to a method of identifying optimal culture condition(s) for trehalose accumulation in a cell or microorganism, which method comprises the steps consisting of:
a) quantifying trehalose in a cell or microorganism which has been cultured under a first environmental condition, by a method of trehalose quantification as described above;
b) quantifying trehalose in the same cell or microorganism which has been cultured under a second environmental condition;
c) repeating step b) as many times as there are environmental conditions to test; and
d) identifying as optimal culture condition(s) that(those) environmental condition(s) under which the highest quantity(ies) of trehalose is(are) detected in the cultured cell or microorganism.

The cell may be an isolated eukaryotic or prokaryotic cell, in particular a cell originating from an animal or a human. Examples of cells include hematopoietic stem cells, embryos, yeasts, or microbial inoculants, in particular those beneficial to agriculture and forestry etc.

An environmental condition is a set of parameters which are usually adjusted by the one skilled in the art for culturing a given cell or microorganism. These parameters typically include nature of the culture medium, pH of the medium, temperature, time length of culture, osmotic pressure, etc. A change in one of these parameters defines a new environmental condition.

The one skilled in the art may rank the environmental conditions tested according to the quantity of trehalose detected for each of them and identify as optimal condition(s) the environmental condition(s) associated with the first four, preferably the first three, more preferably the first two, or still preferably the first one highest trehalose quantity(ies).

### Kits

The invention further provides a kit for qualitatively or quantitatively detecting trehalose in a sample, which kit comprises:
a) a genetically engineered *Pseudomonas fluorescens* strain which comprises a reporter gene inserted in *treA* gene of operon trehalose, wherein *treA* expression is specifically inducible by trehalose, as defined above;
b) optionally appropriate means for detecting reporter gene expression product or activity thereof.

For instance, where the reporter gene is β*-gal,* appropriate means for detecting its expression product (i.e. the enzyme β-galactosidase) may be a substrate liable to be processed by β-galactosidase, and which advantageously produces a detectable signal upon processing thereof. Examples of such substrates include the chromogenic X-gal, or preferably chemiluminescent Galacto-Star substrate from Applied Biosystems.

On the contrary, where the reporter gene is *gfp,* for instance, the kit according to the invention would not contain means of detection since its expression product, the green fluorescent protein, is directly detectable by fluorescence measurement.

In the following figures and examples the nomenclature for the Pyt 5 and Pyt 6 strains is the same as used in the articles Gaballa et al. (1997) and Matthijs et al. (2000).

### FIGURES :

Figure 1 is a representation of trehalose operon and insertion mutants of *Pseudomonas fluorescens* ATCC 17400.

Figure 2 is a representation of the insertion site of the Tn*5*-lacZ2-Km cassette in Pyt 6. Alignments of the sequences of treA-lacZ and Km-treA region of Pyt 6 as named by Gaballa et al. (1997) and Matthijs et al. (2000). Black letters consist in *Pseudomonas fluorescens* ATCC 17400 trehalose operon *treA* gene. Red letters consist in mini-Tn5 lacZ1 and blue letters consist in mini-Tn5 Km. The yellow parts are defined as the respective insertion sites of Tn5 lacZ/Km cassette in Pyt 6.

Figure 3 is a partial sequence of the *treA* gene in BBc6R8 showing a unique restriction site Sphl suitable for a LacZ cassette insertion. The *Sphl* site is located 27 pb upward the insertion site of the lacZ cassette in Pyt 6.

Figure 4 shows the results of assessment of intra- and extra- mycelium trehalose with overlay method. A: *Gaeumannomyces graminis* var. *tritici (Ggt)* with cut or uncut mycelium and control overlay; B: *Ggt* with cut or uncut mycelium and Pyt 6 overlay; C: *Laccaria bicolor (Lb)* with cut or uncut mycelium and control overlay; D: *Lb* with cut or uncut mycelium and Pyt 6 overlay.

Figure 5 is a diagrammatic representation of the quantitative measurement of *treA* induction in Pyt 6 depending of the growth phase (stationary (ST) or exponential (EX) culture), the trehalose concentration in the medium and the addition or not of triton (detergent).

Figure 6 is a diagrammatic representation of OD _{600 nm} measurement of the *P*. *fluorescens* strain Pyt 6 before and after 4h incubation with varying trehalose concentrations. Results shown are mean values from 3 repeated counting. Error bars indicate standard deviation.

Figure 7 shows the results of linear regression analysis between relative values of *treA* induction as derived from the count of luminescence divided with the OD₆₀₀ values after 4 h incubation, and trehalose concentration.

Figure 8 is a diagrammatic representation of the luminescence count as a measure of *treA* induction of Pyt 6 with *Ggt* mycelium extract with or without boiling. The extract derived from 1 g of *Ggt* mycelium cultured for 7 days were tested. For the positive control, 10 ng of purified β-gal and 1 mM trehalose solution with Pyt 6 were assessed. Results shown are mean values from 3 repeated counting. Error bars indicate standard deviations.

Figure 9 is a diagrammatic representation of relative values of luminescence as a measure of *treA* induction of Pyt 6 with *Ggt* mycelium extracts, depending on the test solutions (dilution factor of the mycelium extracts). Results shown are mean values from 3 repeated counting. Error bars indicate standard deviations.

Figure 10 is a diagrammatic representation of measurement of trehalose concentration in *Lb* mycelium extracts diluted to 1/10 and derived from different culture conditions (time culture, temperature, pH, osmotic pressure). Results shown are mean values from 3 repeated counting. Error bars indicate standard deviations.

Figure 11 is a diagrammatic representation of measurement of trehalose concentration in *Ggt* mycelium extracts diluted to 1/10 and derived from different culture conditions (time culture, temperature, pH, osmotic pressure). Results shown are mean values from 3 repeated counting. Error bars indicate standard deviations.

Figure 12 shows the results of measurement of intramycelium trehalose accumulation in different plant-associated fungi and oomycetes.

### EXAMPLES

### Example 1: Influence of lacZ2 cassette insertion in Tre operon on P. fluorescens ATCC 17400 physiology and specific induction by trehalose

Three *lacZ2* insertion mutants in the trehalose operon were tested. Pyt 61 had a weak growth on glucose medium compared to the wild type. The exact insertion site of the *lacZ2* cassette was determined for both *treA⁻* mutants Pyt 5 and Pyt 6. Insertions start respectively, 3104-3111 and 3548-3555 basis downstream the start codon of the *treA* gene in Pyt 5 and Pyt 6 (the first base of the start codon is in position 2616 of AF2298291, SEQ ID NO: 1).

Based on visual assessment of β-galactosidase expression on X-gal medium, Pyt 6 showed *treA* induction only with trehalose 10⁻³ M, whereas Pyt 5 showed also weak induction with maltose. In Pyt 6, *treA* induction by trehalose was not affected by other sugar at higher concentration (Table 1). So, Pyt 6 was chosen as a good candidate to develop the biosensor test in the further study.

Table 1. Influence of added sugars in buffered (pH 7) CAA medium for the β-galactosidase activities of *P*. *fluorescens* ATCC 17400 derived mutants on visual assessment of blue color intensity

| Medium | β-galactosidase activities | |
|---|---|---|
| Added sugar | Pyt 5 | Pyt 6 |
| None | + | - |
| 0.2 % glucose | + | - |
| 2 % glucose | + | + |
| 0.2 % maltose | ++ | - |
| 0.5% maltose | ++ | - |
| 10 mM trehalose | +++ | +++ |
| 0.2 % glucose, 10mM trehalose | +++ | +++ |
| 2 % glucose, 10 mM trehalose | +++ | +++ |
| 0.2 % maltose, 10 mM trehalose | +++ | +++ |
| 0.5% maltose, 10 mM trehalose | +++ | +++ |

| | | |
|---|---|---|
| -; no induction, (+); faintish induction, +; weak induction, ++; highly induction and +++; extremely strong induction | | |

### Example 2: Qualitative detection of trehalose from fungal mycelium

On overlay technique with the adding of X-gal and Pyt 6 was applied for the detection of trehalose from *Ggt* and *Lb* mycelium.

To that end, X-gal - the chromogenic substrate of β-gal - was mixed with a BCAA 0.5% agar medium (Casamino acid 0.5 g, MgSO₄·7H₂O 0.025 g, Agar 2.0 g, H₂O 90 ml sterilized by autoclave at 115°C, 20 min +10 ml of Potassium phosphate buffer (pH 7), +100 µl of 100 mM FeSO₄ solution (final 0.1 mM), 1 ml of 20 % glucose solution (final 0.2 %)), at 60°C and kept at 37°C. Each fungal culture was overlayed with 5 ml of CAA/X-gal medium, with or without the Pyt 6 strain (OD ₆₀₀ₙₘ = 0.1, exponential growth phase), and incubated at 27°C overnight.

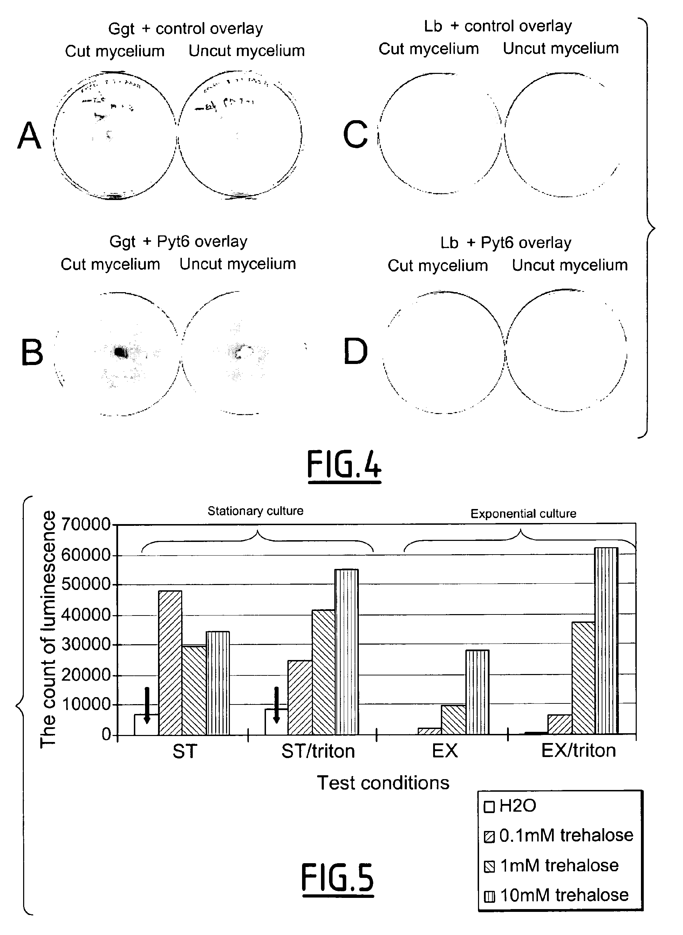

When mycelium was cut just few seconds before pouring the overlay, a dark blue colour for β-galactosidase was observed for both fungi. A control without Pyt 6 in the overlay revealed some residual β-galactosidase activity in cut *Ggt* mycelium but much lower and only in the centre the mycelium colony when compared to the experiment including Pyt 6 in the overlay. No such residual activity was seen for Lb. Both fungi had accumulated detectable trehalose in its mycelium. The same experiment was conducted on uncut growing mycelium but revealed only significative β-galactosidase expression for *Ggt.* Outside mycelium trehalose could be detectable for *Ggt* (Figure 4).

### Example 3: In vitro quantification of trehalose with a biosensor method

Several aims were combined to build a quantitative method of assaying trehalose : reproducibility, miniaturisation, sensitivity and usefulness for small but numerous samples. The test was conducted in microtiter plates that allow bacterial growth assessment to get a relative value of trehalose concentrations. Bacterial treatments were done in the same wells and only 10 µl per well was transferred in black plates for the chemiluminescent reaction. The sensitivity was improved by using a chemoluminescent β-galactosidase detection test (Galacto-Star System, Applied Biosystem).

### ● Influence of P. fluorescens growth phase

One of the first steps was to compare different physiological states of the P. *fluorescens* strain for the relative *treA* induction.

### Preparation of Pyt 6 cells

Pyt 6 strain is pre-cultured in 3 ml liquid BCAA medium (Casamino acid 0.5 g, MgSO₄·7H₂O 0.025 g, H₂O 90 ml sterilized by autoclave at 115°C, 20 min +10 ml of Potassium phosphate buffer (pH 7), 100 µl of 100 mM FeSO₄ solution (final 0.1 mM), 1 ml of 20 % glucose solution (final 0.2 %)), overnight at 27 °C, 200 rpm. 1 ml of the culture is transferred in 2 ml of fresh BCAA medium and incubated at 27°C, 200 rpm. The time length of the culture is adjusted so that the cells are in exponential phase (about 3h culture) or stationary phase (longer culture times, e.g. 8 to 12 hours). The cells are washed twice by centrifugating at 3000 rpm, 5 min, discarding the supernatant, and gently resuspending the bacterial pellet with 3 ml fresh BCAA medium 27°C. The cell suspension is adjusted to OD₆₀₀ = 1.

### Incubation of Pyt 6 cells with sample

100 µl of the suspension of Pyt 6 cells are mixed with 100 µl of a trehalose solution (with concentrations ranging from 0 to 10 mM) or unknown samples in microplate wells. OD_{600 nm} is measured twice in plate spectrophotometer after shaking the plate 30 sec, 2 times. The microtiter plate is then incubated for 4h at 27°C. OD_{600 nm} is measured again twice in plate spectrophotometer after shaking the plate 30 sec.

### Quantification of β-galactosidase activity associated with trehalose-induced treA expression

40 µl of lysis solution (5x Z Buffer (Galacto-Star System; Applied Biosystems)) diluted to 1/5 with ultra pure water and Triton X-100 added up to final 1 %) are added to 200 µl of bacterial suspension in the microplate wells and incubated 30 min at 27 °C with shaking (at 200 rpm). 10 µl of the mixture are transferred into wells of a black microplate and 100 µl of substrate solution (Galacton-Star Substrate 50x diluted to 1/50 with Reaction Buffer Diluent, Applied Byosystems) are added and the mixture is incubated 30 min at 27 °C with shaking (at 200 rpm) in the dark. The light signal is measured in a microtiter luminometer "lumicount" (Packard Instrument Company).

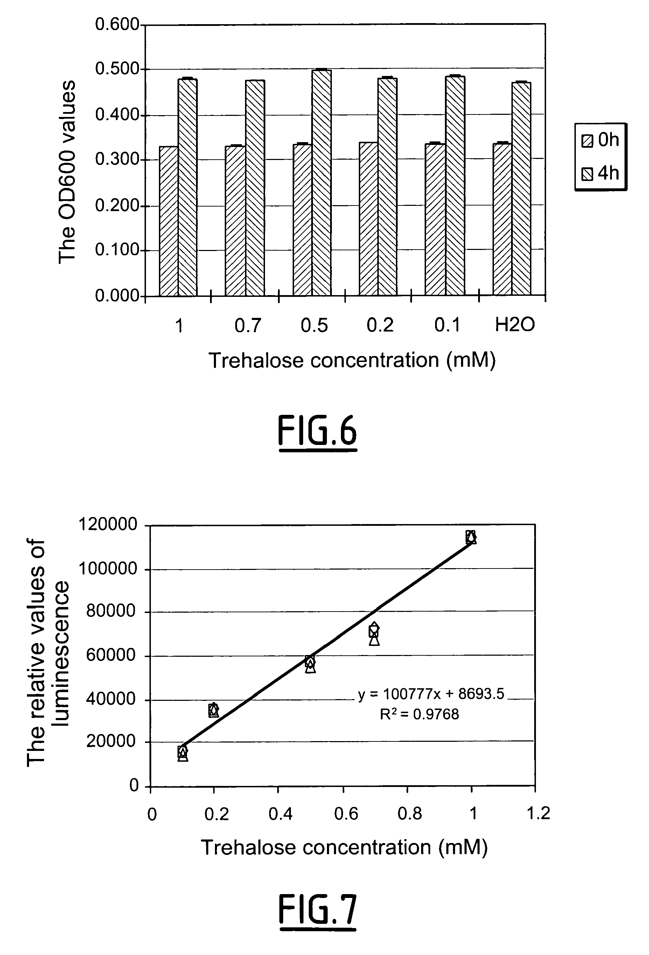

When strains were taken from exponential growth stage the range of specific trehalose detection was higher compared to cells taken from stationary growing phase (Figure 5) and no β-galactosidase induction was observed in absence of trehalose.

Luminescence is quantified for Pyt 6 strain incubated with varying trehalose solutions of varying concentrations (typically 0, 0.1, 0.2, 0.5, 0.7, and 1 mM) to establish standard curves. The relative values of *treA* induction are further derived from the count of luminescence divided with the OD₆₀₀ values after 4 h incubation (correction of the influence of Pyt 6 growth during incubation time - Figure 6). The correspondence between relative luminescence counts and trehalose concentration is shown on Figure 7.

With exponential growth cells, the range of trehalose quantification was from 10⁻⁴ M to 10⁻¹ M.

The use of a glucose enriched CAA buffer medium for bacterial growing and suspension limited the influence of tested solutions. The incubation time with bacterium cells was only four hours to limit growth. No significant difference for cell growth was observed for the different solutions and for the different repeats. The specificity of trehalose induction was demonstrated.

### ● Mycelium trehalose quantification: influence of dilution and boiling

Trehalose was quantified in aqueous extracts from *Ggt* and *Lb* mycelium.

### Preparation of fungal extracts

The fungal extraction was performed as follows. Mycelium are pre-cultured (*Gaeumannomyces. graminis* (*Ggt*): 5 days on PDA (Potato Dextrose 39 g/L, agar 20 g/L); *Laccaria bicolor.* (*Lb*) 10 days on P5 (0.25 g Di NH₄ tartrate, 0.5 g KH₂PO₄, 0.25 g MgSO₄ 7 H₂O, 2.5 g maltose D+, 10 g glucose D+, 0.5 ml thiamine-HCl (100 mg/L), 0.5 ml Kanieltra oligoelement solution (100 ml/L), 10 g agar, 480 ml H2O, adjusted pH to 5.5 with HCl or NaOH). Growing mycelium plugs are explanted to BCAA agar (as defined above) with cellophane membrane or the glass bead/membrane system with liquid BCAA. The cellophane membranes were previously boiled in ultra-pure water + EDTA (372 mg.l-1) during 15 minutes, washed abundantly with ultra-pure water and sterilised by autoclaving twice. Mycelium are cultured for 7 days (*Ggt*) or 14 days (*Lb*), the mycelium are taken with cellophane and dried on a sterile paper to avoid some trace of media, scalped the mycelium with a sterile blade. 1 g of fresh mycelium is weighed into a sterile 1.5 ml eppendorf tube and ground with liquid nitrogen by repetitive dipping the tube into liquid nitrogen and grinding the frozen mycelium with grinder (small stick adapted to 1.5 ml tube). 1 ml of pure sterile water is added before shaking 5 min at room temperature (1 min shake - 1 min relax on ice x 5 cycles), spinning 12000 rpm, 20 min at 4°C. The supernatant is then collected and optionally boiled 5 min at 100°C before storing at - 20°C.

### Quantification of β-galactosidase activity

β-galactosidase activity is quantified as described above with Pyt 6 cells in exponential phase and 4 h incubation with the fungal extracts. The influence of boiling of the fungal extracts on luminescence count was examined. Additionally effect of dilution of the fungal extracts was assessed by using undiluted fungal extracts or extracts diluted to ½ 1/5, 1/10 and 1/20 in 1 mM EDTA.

### Influence of boiling

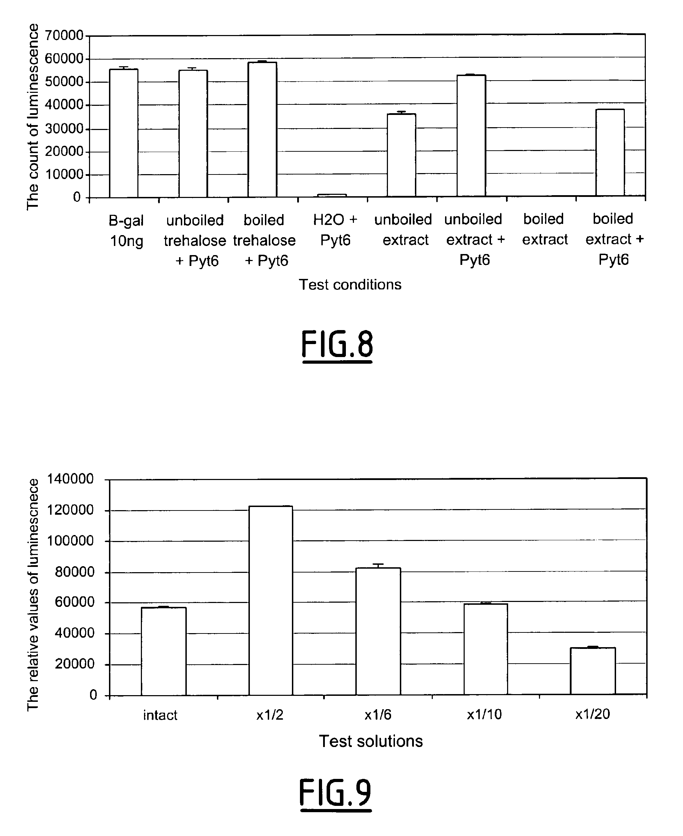

The results of qualitative detection of trehalose from fungal mycelium described in example 2 showed that *Ggt* produces β-galactosidase endogenously. Boiling of the fungal extracts is expected to denature endogenous β-gal and therefore avoid interference with trehalose measurement. Trehalose shows good heat stability. However, it was checked that trehalose is not affected by boiling of the samples (Figure 8). Additionally, it was observed that when *Ggt* mycelium extract (diluted to 1/10) was boiled, no endogenous β-galactosidase activity was ever detected (Figure 8).

This boiling step was applied to give a mycelium blank control and to help the storage of extracts.

### Comparison of trehalose measurement between the biosensor of the invention and NMR method, and influence of dilution

Trehalose measurement were initially performed with undiluted fungal extracts and compared with a classical method of trehalose measurement, i.e. ¹³C natural abundance NMR method (Table 2).

**Table 2: Comparison of trehalose measurement in undiluted fungal extract between the biosensor of the invention and NMR method**

| Mycelium extract | Intramycelial concentration (µM) | |
|---|---|---|
| | Method | |
| | NMR | Biosensor |
| *Ggt* 7 days | 0.42 | 4.22 |
| *Lb* 14 days | 0.52 | 3.36 |

It was hypothesised that the discrepancy observed between the values obtained with the NMR and biosensor methods could be due to the bioavailability of trehalose in the fungal extracts. Accordingly, to assess dilution effects, the bionsensor method was applied to several dilutions of *Ggt* extract. The results of this experiment (Figure 9) showed that the sensibility of the method could be improved by diluting the fungal extract, a 1/10^{th} dilution being chosen for further experiments. Without being linked to this theory, it is thought that the fungal extract could include some inhibition substances for bacterial *treA* induction, which inhibiting effect is withdrawn upon dilution.

Trehalose was quantified in *Ggt* and *Lb* with extracts diluted to 1/10 and the results obtained were compared with measurement obtained with ¹³C natural abundance NMR method (Table 3).

**Table 3: Comparison of trehalose measurement in fungal extracts diluted to 1/10 between the biosensor of the invention and NMR method**

| Mycelium extract | Intramycelial concentration (µM) | |
|---|---|---|
| | method | |
| | NMR | biosensor |
| *Ggt* 7 days | 3.80 | 4.22 |
| *Lb* 14 days | 3.18 | 3.36 |

Thus results of dilution experiments confirmed that the biosensor assay could measure precise amount of trehalose in mycelium. Furthermore, the trehalose concentration data obtained with the biosensor test do not significantly differed from those obtained with the trehalose ¹³C natural abundance NMR method.

### Example 4: Effect of different environmental factors on trehalose accumulation in Ggt and Lb mycelium

Different culture conditions with varying culture time, temperature, pH and osmotic pressure were assayed for *Ggt* and *Lb* Influence of these varying conditions on trehalose accumulation in mycelium was determined with Pyt 6 by measuring β-gal activity. Since trehalose is regarded as a protectant for storage of cells, tissues or microorganisms, identifying culture conditions enabling optimal trehalose accumulation could allow prepare cultures with improved preservation properties.

Ggt and Lb were cultured as described above, except for the following parameters:
- Culture time: *Ggt* 7, 14, and 21 days,
   *Lb* 14, 21, and 28 days,
   in CAA medium, pH 7.0 at 20 °C.
- Temperature: 5, 10, 15 and 20 °C in CAA medium, pH 7.0 for 7 days *(Ggt)* or 14 days (Lb).
- pH: 5.5, 7.0 and 8.0 in CAA medium for 7 days (*Ggt*) or 14 days (*Lb*)*.*
- Osmotic pressure: 0, 5, 10 and 20 % PEG-3350 in CAA medium, pH 7.0 for 7 days *(Ggt)* or 14 days (Lb).

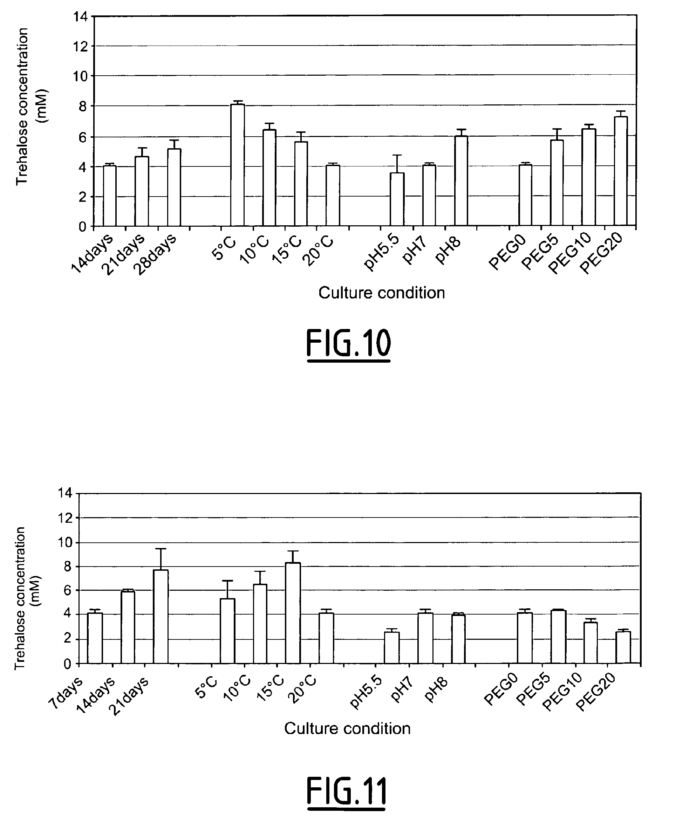

The results observed for *Lb* and *Ggt* culture are shown on Figures 10 and 11, respectively.

In both fungi, trehalose was more accumulated with aging. This effect is more pronounced with *Ggt.* Colder growth temperature increased trehalose concentration in *Lb* mycelium compared to warmer temperature. For *Ggt,* there was an optimum temperature for high trehalose accumulation. High pH values increased trehalose concentration in Lb mycelium when pH did not influence trehalose accumulation in *Ggt.* A low osmotic pressure increases trehalose concentration in *Lb* mycelium. In *Ggt,* there was no variation in trehalose accumulation with different osmotic pressures.

In view of these results it seems that the optimal culture conditions for trehalose accumulation would be unfavourable growth conditions : 28 day culture, 5°C, pH 8 and low osmotic pressure for *Lb,* and 21 day culture, 15°C, pH 7 and high osmotic pressure for *Ggt.*

### Example 5: Measurement of trehalose accumulation in different fungi and oomycetes

The developed biosensor method was applied for screening of microorganism naturally accumulating trehalose.

The Fungi tested were Lb c: *Laccaria bicolor* (carpophore), BR32 : *Magnaporthe grisea ,* Guy11 : *Magnaporthe grisea* MP : *Mycosphaerella pinodes,* Ggt26 : *Gaeumannomyces graminis* var. *tritici,* Ggt17 : *Gaeumannomyces graminis* var. *tritici,* Phoma86 : *Phoma foveata,* Lb m : *Laccaria bicolor* (mycelium), PH : *Phialophora radicicola* var. *radicicola,* Phoma88 : *Phoma exigua* var. *exigua ,* GA9 : *Gaeumannomyces graminis* var. *avenae,* Mp u *: Melampsora larici-populina* (uredia), and Mp t : *Melampsora larici-populina* (telia).

The Oomycetes tested were AE5 : *Aphanomyces euteiches,* RB 84 : *Aphanomyces euteiches,* Pv : *Pythium violae,* Ps : *Pythium sulcatum,* PM8 : *Phytophthora megasperma,* and Pi : *Phytophthora infestans.*

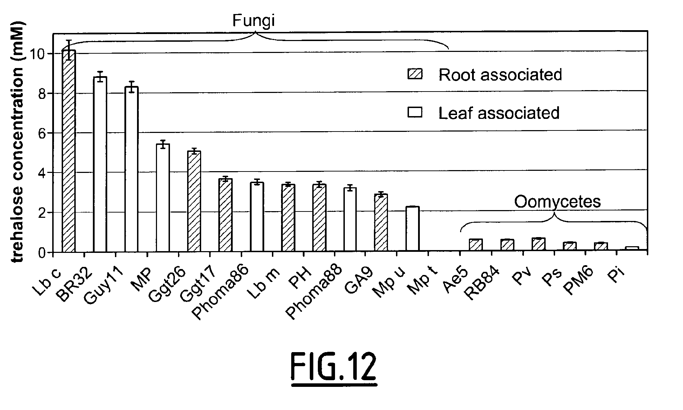

*Magnaporthe grisea* and *Ggt* are the most accumulating trehalose mycelia. Besides, no or trace of trehalose is detectable in oomycetes like *Pythium ultimum* and *Aphonomyces euteiches* (Figure 12).

### Example 6 : Trehalose assay in high-throughput format

From the knowledge derived from the use of the qualitative and quantitative methods, the inventors have developed a semi-quantitative method that allows a high throughput pre-screening of the presence of trehalose in liquid samples. The invention helps also to the check quickly the quality of controls without trehalose before applying the quantitative method. The semi-quantitative method combines a direct colorimetric observation with the use of the chromogenic X-Gal and the conditions of Pyt 6 culture derived from the quantitative method. The detection limit is 0.5 mM trehalose in 10 µl of the tested liquid sample (biological or other extracts). With the semi-quantitative method, there was no visible induction with the sugars (2 %) like glucose, maltose, arabinose, galactose, fructose and glycerol.

This method was carried out according to the following steps :
- preculture of Pyt 6 to exponential growth in the BCAA medium at 27°C,
- distribution of 190 µl of this Pyt 6 suspension at OD 600 nm = 0.1 in wells of a 96 well microtiter plate ,
- incorporation of 2 µl X-Gal (20 mg/ml) in each well,
- incorporation of 10 µl of a standard range of trehalose (0.1 mM to 2 mM trehalose) or 10 µl of tested samples or 10 µl of pure water or culture medium for the blank control,
- incubation during 18 hours at 150 rpm at 27°C,
- comparative observation of the intensity of the blue color in wells,
- the lower detection limit is 0.5 mM trehalose in the tested sample.

### REFERENCES

Araujo PS, Panek AC, Ferreira R, Panek AD. Determination of trehalose in biological samples by a simple and stable trehalase preparation. Anal Biochem. 1989 Feb 1;176(2):432-6.

Arguelles JC. 2000 Physiological roles of trehalose in bacteria and yeasts: a comparative analysis. Arch Microbiol. 2000 Oct;174(4):217-24. Review. Erratum in: Arch Microbiol 2000 Dec;174(6):456.

Attfield PV. 1987 Trehalose accumulates in Saccharomyces cerevisiae during exposure to agents that induce heat shock response. FEBS Lett. 1987 225(1-2):259-63.

Blazquez MA, Santos E, Flores CL, Martinez-Zapater JM, Salinas J, Gancedo C. 1998 Isolation and molecular characterization of the Arabidopsis TPS1 gene, encoding trehalose-6-phosphate synthase. Plant J. 13(5):685-9

Bellinger Y, Larher F. 1988 A 13C comparative nuclear magnetic resonance study of organic solute production and excretion by the yeasts Hansenula anomala and Saccharomyces cerevisiae in saline media. Can J Microbiol. 34(5):605-12

Brodmann A, Schuller A, Ludwig-Muller J, Aeschbacher RA, Wiemken A, Boller T, Wingler A. 2002 Induction of trehalase in Arabidopsis plants infected with the trehalose-producing pathogen Plasmodiophora brassicae. Mol Plant Microbe Interact. 15(7):693-700

Boos W, Ehmann U, Forkl H, Klein W, Rimmele M, Postma P. 1990 Trehalose transport and metabolism in Escherichia coli. J Bacteriol. 172(6):3450-61.

Cataldi TRI, Campa C, Angelotti M, Bufo SA. 1999 Isocratic separations of closely-related mono- and disaccharides by high-performance anion-exchange chromatography with pulsed amperometric detection using dilute alkaline spiked with barium acetate. J Chromatography A. 855: 539-550.

Chapon, A., A.-Y. Guillerm, L. Delalande, L. Lebreton, and A. Sarniguet. 2002 Dominant colonisation of wheat roots by Pseudomonas fluorescens Pf29A and selection of the indigenous microflora in the presence of the take-all fungus. European Journal of Plant Pathology 108:449-459

Chen F, Nakamura T, Wada H. (2004) Development of new organ preservation solutions in Kyoto University. Yonsei Med. J. ;45(6):1107-14.

Colaco C, Sen S, Thangavelu M, Pinder S, Roser B. 1992 Extraordinary stability of enzymes dried in trehalose: simplified molecular biology. Biotechnology. 10(9):1007-11.

Carninci P, Nishiyama Y, Westover A, Itoh M, Nagaoka S, Sasaki N, Okazaki Y, Muramatsu M, Hayashizaki Y. 1998 Thermostabilization and thermoactivation of thermolabile enzymes by trehalose and its application for the synthesis of full length cDNA. Proc Natl Acad Sci U S A. 95(2):520-4.

Dijkema C, Kester HC, Visser J. 1985 13C NMR studies of carbon metabolism in the hyphal fungus Aspergillus nidulans. Proc Natl Acad Sci U S A. 82(1):14-8.

Elbein AD, Pan YT, Pastuszak I, Carroll D. 2003 New insights on trehalose: a multifunctional molecule. Glycobiology. 13(4):17-27.

Eroglu A, Toner M, Toth TL. 2002 Beneficial effect of microinjected trehalose on the cryosurvival of human oocytes. Fertil Steril. 77(1): 152-8.

Fahy P.C. and Lloyd A.B. 1983 The fluorescent Pseudomonas. In Plant bacterial disease : a diagnostic guide (Fahy P.C. and Persley G.S. Editors) p145, Academic Press, London

Fillinger S, Chaveroche MK, van Dijck P, de Vries R, Ruijter G, Thevelein J, d'Enfert C. 2001 Trehalose is required for the acquisition of tolerance to a variety of stresses in the filamentous fungus Aspergillus nidulans. Microbiology. 147:1851-62.

Frey-Klett P., Pierrat J.C. et Garbaye J. 1997. Location and survival of mycorrhiza helper Pseudomonas fluorescens during establishment of ectomycorrhizal symbiosis between Laccaria bicolor and Douglas fir. Applied and Environmental Microbiology. 63:139-144.

Gaballa A, Abeysinghe PD, Urich G, Matthijs S, De Greve H, Cornelis P, Koedam N. 1998 Trehalose induces antagonism towards Pythium debaryanum in Pseudomonas fluorescens ATCC 17400. Appl Environ Microbiol. 1997 Nov;63(11):4340-5. Erratum in: Appl Environ Microbiol 1998 Apr;64(4):1587. Appl Environ Microbiol 1998 Sep;64(9):3547.

Garbaye, J. and Duponnois, R., 1992. Specificity and function of mycorrhization helper bacteria (MHB) associated with the Pseudotsuga menziesii×Laccaria laccata symbiosis. Symbiosis 14, pp. 335-344.

Hansen LH, Sorensen SJ. 2001 The use of whole-cell biosensors to detect and quantify compounds or conditions affecting biological systems. Microbial Ecology. 42 : 483-494.

Helfert C, Gotsche S, Dahl MK. 1995 Cleavage of trehalose-phosphate in Bacillus subtilis is catalysed by a phospho-alpha-(1-1)-glucosidase encoded by the treA gene. Mol Microbiol. 16(1):111-120.

Igashiyama T. 2002 Novel functions and applications of trehalose. Pure Appl. Chem., Vol. 74, No. 7, pp. 1263-1269.

Keen, N. et P. Williams. 1969. Translocation of sugars into infected cabbage tissues during clubroot development. Plant Physiol. 44: 748-754

Jaeger CH 3rd, Lindow SE, Miller W, Clark E, Firestone MK. 1999 Mapping of sugar and amino acid availability in soil around roots with bacterial sensors of sucrose and tryptophan. Appl Environ Microbiol. 65(6):2685-90.

Jensen JB, Peters NK, Bhuvaneswari TV. 2002 Redundancy in periplasmic binding protein-dependent transport systems for trehalose, sucrose, and maltose in Sinorhizobium meliloti. J Bacteriol. 184(11):2978-86

Kandror O, DeLeon A, Goldberg AL. Trehalose synthesis is induced upon exposure of Escherichia coli to cold and is essential for viability at low temperatures. Proc Natl Acad Sci U S A. 2002 99(15):9727-32.

Kienle I, Burgert M, Holzer H. 1993 Assay of trehalose with acid trehalase purified from Saccharomyces cerevisiae. Yeast 9(6):607-11.

Kaushik and Bhat 2003 Why is trehalose an exceptional protein stabilizer?: An analysis of the thermal stability of proteins in the presence of compatible osmolyte trehalose. J. Biol Chem. 278:26458-26465.

Leveau, J.H.J. et Lindow, S.E. 2002. Bioreporters in microbial ecology. Current Opinion in Microbiology, 5: 259-265.

Martin, F., D. Canet et J.P. Marchal. 1984. In vivo natural abondance 13C NMR studies of the carbohydrate storage in ectomycorrhizal fungi. Physiol. Veg. 22:733-743

Matthijs S, Koedam N, Cornelis P, De Greve H. 2000 The trehalose operon of Pseudomonas fluorescens ATCC 17400. Res Microbiol. 151 (10):845-51.

Mellor, R.B. 1988 Distribution of trehalase in soybean root nodule cells: implications for trehalose metabolism. J. Plant. Physiol. 133:173-177.

Meikle AJ, Chudek JA, Reed RH, Gadd GM. 1991 Natural abundance 13C-nuclear magnetic resonance spectroscopic analysis of acyclic polyol and trehalose accumulation by several yeast species in response to salt stress. FEMS Microbiol Lett. 66(2):163-7.

Miller WG, Brandl MT, Quinones B, Lindow SE. Biological sensor for sucrose availability: relative sensitivities of various reporter genes. Appl Environ Microbiol. 2001 Mar;67(3):1308-17.

Muller J, Boller T, Wiemken A. 2001 Trehalose becomes the most abundant non-structural carbohydrate during senescence of soybean nodules. J Exp Bot. 52(358):943-7

Paiva CLA, Panek, AD Biotechnological applications of the disaccharide trehalose. Biotechnoly Annual Review 1996 (El-Gewely MR, ed.) vol.2 pp.293-314 Elsevier Science

Shi L, Guttenberger M, Kottke I, Hampp R. 2002 The effect of drought on mycorrhizas of beech (Fagus sylvatica L.): changes in community structure, and the content of carbohydrates and nitrogen storage bodies of the fungi. Mycorrhiza. 12(6):303-11.

Scheinkonig C, Kappicht S, Kolb HJ, Schleuning M. 2004 Adoption of long-term cultures to evaluate the cryoprotective potential of trehalose for freezing hematopoietic stem cells. Bone Marrow Transplant.; 34(6):531-6.

Standing D; Meharg AA; Killham K. (2003) A tripartite microbial reporter gene system for real-time assays of soil nutrient status. FEMS Microbiol. Letters 220(1), 35-39

Streeter JG. 1985 Accumulation of alpha,alpha-trehalose by Rhizobium bacteria and bacteroids. J Bacteriol. 1985 164(1):78-84

Vogel G, Aeschbacher RA, Muller J, Boller T, Wiemken A. 1998 Trehalose-6-phosphate phosphatases from Arabidopsis thaliana: identification by functional complementation of the yeast tps2 mutant. Plant J. 13(5):673-83.

Wingler A. 2002 The function of trehalose biosynthesis in plants Phytochemistry. 60:437-40

## Claims

1. Use of a genetically engineered *Pseudomonas fluorescens* strain comprising a reporter gene inserted in *treA* gene of operon trehalose, and wherein *treA* expression is specifically inducible by trehalose, for assaying trehalose in a sample.

2. The use according to claim 1, wherein said reporter gene is inserted in a region homologous to nucleotides 3498 to 3605 of SEQ ID NO: 1.

3. The use according to claim 1 or 2, wherein said P. *fluorescens* strain is selected from the group consisting of ATCC 17400, BBc6R8 and Pf29 strains.

4. The use according to any of claims 1 to 3, wherein *treA* expression is not inducible by glucose and/or maltose.

5. The use according to claim 4, wherein treA expression is further not inducible by arabinose and/or fructose and/or galactose and/or saccharose.

6. The use according to any of claims 1 to 5, wherein genetically engineered *P*. *fluorescens* strain is selected from the group consisting of Pyt 6 deposited before CNCM on February 10, 2006, under number I-3571, and a BBc6R8 strain in which a reporter gene is inserted between nucleotides 1362 and 1363 of SEQ ID NO:2.

7. A genetically engineered *Pseudomonas fluorescens* strain comprising a reporter gene inserted in *treA* gene of operon trehalose, as defined above, and wherein said reporter gene is inserted in a region of *treA* gene homologous to nucleotides 3498 to 3605 of SEQ ID NO:1, with the provisio that said *P*. *fluorescens* strain is not Pyt 6.

8. A genetically engineered *P*. *fluorescens* strain according to claim 7, wherein said *P*. *fluorescens* strain is selected from the group consisting of ATCC 17400, BBc6R8 and Pf29 strains.

9. A genetically engineered P. *fluorescens* strain according to claim 7 or 8, wherein said *P*. *fluorescens* strain is a BBc6R8 strain in which a reporter gene is inserted between nucleotides 1362 and 1363 of SEQ ID NO: 2.

10. A method of qualitatively detecting trehalose in a sample, said method comprising the steps consisting of:
a) contacting said sample with a genetically engineered *Pseudomonas fluorescens* strain as defined in any of claims 1 to 9; and
b) detecting presence and/or activity of an expression product of the reporter gene;
wherein presence and/or activity of the expression product of the reporter gene is indicative of the presence of trehalose in the sample.

11. A method according to claim 10 wherein the sample is selected from the group consisting of foods, beverages, microorganisms, eukaryotic cells and tissues, solutions for preservation of living tissues or cells and any ingredient containing trehalose.

12. A method of quantifying trehalose in a sample which comprises the steps consisting of:
a) contacting said sample with a genetically engineered *Pseudomonas fluorescens* strain as defined in any of claims 1 to 9; and
b) measuring level and/or activity of an expression product of the reporter gene; and
c) comparing the level and/or activity measured in step b) with the level and/or activity of the expression product of the reporter gene elicited by a range of trehalose standards to determine trehalose concentration in the sample.

13. The method according to claim 12, wherein said genetically engineered *P. fluorescens* strain is in the form of a suspension of cells in exponential growth phase.

14. The method according to claim 12 or 13, wherein said genetically engineered *P*. *fluorescens* strain is Pyt 6.

15. The method according to claim 14, wherein step a) is performed by mixing the sample with a suspension of Pyt 6 cells in a buffered medium with a pH ranging from 6.9 to 7.2, and incubating the mixture at a temperature ranging from 20 to 27°C, for a time length of 3 to 4 hours.

16. The method according to any of claims 12 to 15, wherein the sample is a microorganism or any liquid sample thereof.

17. The method according to any of claims 12 to 16, wherein the sample is a fungal mycelium extract which has been boiled and diluted before contacting with said genetically engineered *P*. *fluorescens* strain.

18. A method of screening trehalose accumulating microorganisms, which method comprises the steps consisting of:
a) assaying trehalose in candidate microorganisms by a method according to any of claims 10 to 17; and
b) identifying as microorganisms accumulating trehalose those in which trehalose is detected.

19. A method of screening trehalose accumulating microorganisms useful for producing trehalose on industrial scale which method comprises the steps consisting of:
a) quantifying trehalose in candidate microorganisms by a method according to any of claims 12 to 17; and
b) identifying as useful for producing trehalose on industrial scale that(those) microorganism(s) in which the highest quantity(ies) of trehalose is(are) detected.

20. A method of identifying optimal culture condition(s) for trehalose accumulation in a cell or microorganism, which method comprises the steps consisting of:
a) quantifying trehalose in a cell or microorganism which has been cultured under a first environmental condition, by a method according to any of claims 12 to 17;
b) quantifying trehalose in the same cell or microorganism which has been cultured under a second environmental condition;
c) repeating step b) as many times as there are environmental conditions to test; and
d) identifying as optimal culture condition(s) that(those) environmental condition(s) under which the highest quantity(ies) of trehalose is(are) detected in the cultured cell or microorganism.

21. A kit for qualitatively or quantitatively detecting trehalose in a sample, which kit comprises:
a) a genetically engineered *Pseudomonas fluorescens* strain as defined in any of claims 1 to 9; and
b) optionally appropriate means for detecting reporter gene expression product or activity thereof.
